# EUROPÄISCHE PATENTSCHRIFT

(11) **EP 0 555 724 B1**
(45) Veröffentlichungstag und Bekanntmachung des Hinweises auf die Patenterteilung: **14.01.1998**
(21) Anmeldenummer: 93101556.4
(22) Anmeldetag: 02.02.1993
(51) Int. Cl.: A61L 11/00

(54) **Verfahren und Vorrichtung zum Desinfizieren von Abfällen**
Method and device for the disinfection of waste
Méthode et dispositif pour la désinfection de déchets

(30) Priorität: 14.02.1992 DE 4204444
(43) Veröffentlichungstag der Anmeldung: 18.08.1993
(73) Patentinhaber: KEG Sonderabfall-Entsorgungsgesellschaft mbH, D-13597 Berlin (DE)
(72) Erfinder: Birkholz, Michaela, W-1000 Berlin 28 (DE); Drauschke, Stefan, Dr., W-1000 Berlin 10 (DE); Hörber, Gerhard, Dr., W-1000 Berlin 49 (DE)
(74) Vertreter: Alber, Norbert

(56) Entgegenhaltungen:
- EP-A- 0 031 790
- EP-A- 0 454 122
- DE-A- 3 444 197
- US-A- 4 166 096

## Beschreibung

In der Industrie und vor allem auch in Krankenhäusern fallen infektiös kontaminierte Materialien an, für deren Beseitigung der Gesetzgeber hohe Anforderungen vorschreibt.

Bei den Krankenhausabfällen wird dabei zwischen sogenannten "nassen" Abfällen wie etwa Blut und Organteilen etc. und sogenannten "trockenen" Abfällen wie etwa benutztem Verbandmaterial, Papierwäsche, Tupfer etc. unterschieden.

Werden diese Materialien von jedem Krankenhaus aus Sicherheitsgründen einzeln vor Ort desinfiziert oder sterilisiert, so verursacht dies sehr hohe Kosten, da die hierfür notwendige Anlage durch die entsprechenden Abfälle nur eines Krankenhauses in der Regel nicht ausgelastet werden kann.

Die Erfindung bezieht sich daher auf ein Verfahren sowie eine Vorrichtung zur Durchführung dieses Verfahrens zum Entsorgen solcher infektiöser, trockener Abfälle, ohne daß die dafür notwendigen Einrichtungen bei jedem nutzenden Krankenhaus zur Verfügung gestellt werden müssen.

Prinzipiell werden diese trockenen Abfälle in den Krankenhäusern in speziellen Abfallsäcken gesammelt und in verschließbaren zentralen Containern deponiert. Die Desinfektion bzw. Sterilisation erfolgt in an sich bekannter Weise in druckdichten, mit Dampf beaufschlagbaren Autoklaven nach dem sogenannten fraktionierten Vakuum-Dampfverfahren.

Dabei wird der mit dem infizierten Material gefüllte Behälter jeweils mehrmals nacheinander mit Wasserdampf gefüllt und anschließend weitestgehend evakuiert. In einem letzten Schritt wird der Autoklav mit Sattdampf mit Überdruck beaufschlagt und dieser Zustand bei einer Dampftemperatur von ca. 110°C etwa 15 Minuten gehalten, wodurch der Inhalt des Autoklaven desinfiziert wird. Bei einer Haltetemperatur von 134°C des Sattdampfes erfolgt darüber hinaus eine Sterilisation. Die speziellen Abfallsäcke, die ohne erneutes Öffnen mitsamt ihren infektiösen Inhaltsstoffen in den Autoklaven geworfen werden, sind wasserdampfdurchlässig, so daß der gesamte Inhalt des Autoklaven die Desinfizierung bzw. Sterilisation vollzieht.

Um den einzelnen Krankenhäusern die eben beschriebene Entsorgung ohne die notwendigen baulichen Anlagen zu ermöglichen, ist es gemäß der DE-OS 30 39 173 bereits bekannt, auf einem LKW montierte, mobile Autoklaven zu verwenden, die beim Krankenhaus mit den entsprechenden Abfällen beladen werden und zu einer feststehenden Entsorgungsstation gefahren werden, wo der Autoklav über entsprechende Anschlüsse mit Prozeßdampf gefüllt und in seinem doppelwandigen Mantelraum auch beheizt sowie über Absauganschlüsse die Atmosphäre des Autoklaven abgesaugt werden kann.

Nachteilig ist dabei, daß diese Entsorgungsstation aufgrund des relativ hohen baulichen Aufwandes nicht beliebig oft erstellt werden kann, so daß die Autoklaven-Fahrzeuge weite Wege zwischen Krankenhäusern und Entsorgungsstation zurückzulegen haben, was nicht nur die Kosten erhöht, sondern trotz der sicheren Bauweise der Autoklaven auch das Unfallrisiko.

Zusätzlich hat das Problem der Desinfektion der bei den ersten Evakuierungsschritten abgesaugten Atmosphäre des Autoklaven bisher einen zu hohen Aufwand erfordert und ebenso das Problem der Geruchsbelästigung, welches zusätzlich zu den technischen Anforderungen an derartige Entsorgungsstationen eine weitere Einschränkung der Standortwahl für solche Entsorgungsstationen mit sich brachte.

Es ist daher die Aufgabe gemäß der vorliegenden Erfindung, ein Verfahren zur Beseitigung infektiöser Abfälle, sowie eine Vorrichtung zu dessen Durchführung, zu schaffen, welches hinsichtlich Desinfizierung und Geruchsneutralisierung der Abluftbrüden unproblematisch ist und in örtlicher Hinsicht einen variablen Einsatz dieser Entsorgungsart ermöglicht.

Diese Aufgabe wird durch die kennzeichnenden Merkmale der Ansprüche 1, 3 und 11 gelöst. Vorteilhafte Ausführungsformen ergeben sich aus den Unteransprüchen.

Erfindungsgemäß wird das Verfahren zum Desinfizieren mittels fraktioniertem Vakuum-Dampfverfahren dahingehend weitergebildet, daß bei der Evakuierung nach den Normaldruck-Dampfphase die Dampfbrüden, die kontaminiert sind, zunächst von allen Stoffen gereinigt werden, die gleich groß oder größer sind als ein Virus, eine Bakterie oder ein anderer Keim. Diese Reinigung geschieht mit Hilfe von mehreren, vorzugsweise zwei, in Reihe geschalteten Filtern mit abnehmender Durchlaßgröße, wobei der feinere eine Durchlaßgröße von 0,2 µm besitzt, so daß in diesem sogenannten Sterilfilter auch Keime hängenbleiben. Ein so feiner Sterilfilter würde natürlich von Dampftröpfchen sofort verstopft werden, da der enthaltene Dampf beim Durchlaufen des Filters teilweise kondensiert und die sich dabei bildenden Wassertröpfchen sofort die Poren des Sterilfilters verstopfen würden. Der dem Sterilfilter vorgeschaltete Vorfilter mit etwa 1,2 µm ist daher fein genug, um Dampftröpfchen zurückzuhalten. Das an den Filtern anfallende Kondensat wird nach einer Desinfektion separat ins Abwasser abgeleitet.

Die Filter können mittels Prozeßdampf, der nicht kontaminiert ist, gereinigt werden. Zu diesem Zweck wird der gröbere Vorfilter gegen die normale Durchlaufrichtung beaufschlagt, wodurch die in ihm festgesetzten Teilchen in den in den Sumpf des Vorfilters zurückgedrückt werden. Der Sterilfilter und auch der Vorfilter kann in Durchlaufrichtung mit Prozeßdampf beaufschlagt und durch entsprechende Haltezeit der Prozeßdampf-Einwirkung desinfiziert werden.Dabei ist davon auszugehen, daß kaum Keime den Sterilfilter erreichen werden, da sich die Keime in der Regel an in der Abluft enthaltenen feinsten Wassertröpfchen anlagern und damit zusammen mit diesen Wassertröpfchen bereits im Vorfilter hängenbleiben.

Zu diesem Zweck werden die zwischen dem Autoklaven und den Filtern ggfs. angeordneten Ventile geöffnet und die in Durchlaufrichtung nach den Filtern angeordneten Ventile geschlossen, während der Innenraum des Autoklaven mit Dampf beaufschlagt wird. Dadurch wird auch der Inhalt der Filter sowie der Sumpf der Filter zusammen mit dem Inhalt des Autoklaven während der abschließenden Überdruck-Dampfphase sterilisiert bzw. desinfiziert.

Für das Entfernen des nicht regenerierbaren Sterilfilters wird dieser vor Entfernen nochmals zusätzlich zur Sicherheit mittels Prozeßdampf beaufschlagt, indem Prozeßdampf mittels einer Bypass-Leitung direkt zu den Filtern geleitet und die umgebenden Ventile geschlossen werden.

Nach Durchlaufen der Filter werden die - nun keimfreien - Abluftbrüden über einen Kondensator geführt um einen möglichst hohen Anteil des enthaltenen Dampfes zu kondensieren und das Kondenssat ebenfalls dem Abwasser zuzuführen. Die weitgehend trockene Abluft wird über eine Anlage zur Geruchsneutralisierung an die Umgebungsluft abgeführt.

Anders wird mit den Abluftbrüden verfahren, die beim Evakuieren des Autoklaven nach der Überdruck-Dampfphase abgesaugt werden: Nach dem Ablassen des Innenraum-Druckes von einem im oberen Bereich angeordneten Absaugpunkt wird vom tiefsten Punkt des Innenraums des Autoklaven das dort angesammelte Kondensat abgepumpt, wobei die immer enthaltenen gasförmigen Bestandteile z.B. mittels einem Zyklon abgetrennt und nach Geruchsneutralisierung an die Umgebungsluft abgegeben werden. Die flüssigen Bestandteile werden nach Abkühlung auf annähernd Umgebungstemperatur dem Abwasser zugeführt.

Nach dem Abpumpen des Kondensats wird die im wesentlichen gasförmige Atmosphäre aus dem Autoklaven entfernt, indem entweder von diesem tiefsten Punkt aus nach Beseitigung des Kondensates weiter abgesaugt wird, oder im weiteren Verlauf von einem höherliegenden Absaugpunkt aus die Atmosphäre aus dem Autoklaven abgesaugt wird.

In jedem Fall werden diese Abluftbrüden über einen Kondensator geleitet und nach Flüssiganteil und Gasanteil getrennt dem Abwasser bzw. der Umgebungsluft zugeführt. Zusätzlich können die Abluftbrüden über Filter geleitet werden, um vor allem Feststoffe herauszufiltern, die die Vakuumpumpen beschädigen könnten.

Um während der Haltezeit in der Überdruck-Dampfphase die Temperatur im Autoklaven nicht absinken zu lassen, ist dieser in an sich bekannter Weise doppelwandig ausgebildet und in dem den Innenraum umgebenden Mantelraum ebenfalls mit Prozeßdampf beaufschlagt. Der Prozeßdampf wird gesättigt gehalten, indem das in den Zuführleitungen für den Prozeßdampf und im Mantelraum des Autoklaven anfallende Kondensat in einem Kondensatsammler zwischengespeichert und über eine entsprechende Pumpe dem Prozeßdampf wieder zugeführt wird. Für den Fall, daß keine ausreichende Kondensatmenge vorliegt, kann die Sättigung auch mit Frischwasser erfolgen.

Die Geruchsneutralisierung der Abluftbrüden erfolgt, indem die Gase zunächst durch eine Zone aus feinstem, beispielsweise durch Ultraschall-Düsen erzeugten Nebel aus einer Waschflüssigkeit geführt werden, in der sich die Geruchsstoffe an den Nebeltröpfchen anlagern. In einer anschließenden Abscheideschicht aus einem Feststoff mit großer spezifischer Oberfläche wie etwa Lavagestein, durch die die Gase im Gegenstrom zur auf die Abscheideschicht aufgesprühten Umlaufflüssigkeit geführt werden, erfolgt die Trennung der mit Geruchsstoffen beladenen Flüssigkeitströpfchen von den abzuführenden Gasen. Die noch nicht zurückgehaltenen Tröpfchen werden anschließend in einem konventionellen Tropfenabscheider zurückgehalten. Die Waschflüssigkeit wird dabei im Kreislauf geführt und unterliegt somit einer zunehmenden Verschmutzung, wobei lediglich bei der Feinstnebel-Erzeugung unverschmutzte Waschflüssigkeit verwendet wird.

Die Waschflüssigkeit auf wässeriger Basis wird anschließend als Umlaufflüssigkeit verwendet und kann Lösungsvermittler enthalten, um die schwer wasserlöslichen Luftinhaltsstoffe auszuwaschen, wie etwa ätherische Öle, und ggfs. zusätzlich Substanzen, die eine chemische Reaktion mit den geruchsintensiven Luftinhaltsstoffen eingehen, wodurch ein Desodorierungseffekt stattfindet. Hierfür kommen z.B, Oxidationsmittel wie Wasserstoff-Peroxid in Frage.

Die Geruchsneutralisierung kann alternativ auch mittels eines Adsorptionsfilters vorgenommen werden, indem die Abluftbrüden z.B. über trockene Aktivkohle geleitet wird.

Der fahrbar ausgebildete Autoklav weist dabei neben dem eigentlichen, doppelwandigen Autoklaven lediglich die Anschlüsse für die Dampfbeaufschlagung des Innenraumes und des Mantelraumes auf, sowie einen Entnahmeanschluß für das im Mantelraum gebildete Kondensat und wenigstens einen Absauganschluß für die Atmosphäre im Innenraum des Autoklaven.

Alle anderen für das oben beschriebene Verfahren notwendigen Aggregate, Leitungen und Ventile wie Filter, Kondensator, Flüssigabscheider, Gaswäscher, Kondensatsammler und Steuereinheit sind in einer sogenannten Kraftstation untergebracht, die ebenfalls mobil ausgebildet sein kann durch Unterbringung in einem leicht transportablen Norm-Container. In der Regel wird diese Kraftstation wiederum externe Anschlüsse vor allem für Prozeßdampf und elektrische Energie besitzen, da nach Möglichkeit Dampf aus Überschußkapazitäten von Heizkraftwerken etc. benutzt wird anstelle der wesentlich teueren Erzeugung von Prozeßdampf aus Primärenergie.

Die relativ mobile Ausgestaltung der Kraftstation gibt jedoch die Möglichkeit, diese Kraftstation in größeren zeitlichen Abständen von einem geeigneten Aufstellungsort zu einem anderen zu transportieren, und dadurch die Anfahrwege der mobilen Autoklaven stark zu verringern.

Ferner enthält die Kraftstation einen Frischwasser-Anschluß zur Sättigung des Prozeßdampfes und Verdünnen der Umlaufflüssigkeit im Gaswäscher. Um die Kapazität des notwendigen Frischwasser-Anschlusses gering zu halten, sind sowohl der Kondensator als auch eventuell zusätzliche Kühleinrichtungen an den Flüssigkeitsabscheidern, mit deren Hilfe die Flüssigbestandteile auf annähernd Umgebungstemperatur heruntergekühlt werden, um sie dem normalen Abwasser zuführen zu dürfen, luftgekühlt. Zum Zwecke dieser Abkühlung sind vorzugsweise zwei getrennte Flüssigkeitsabscheider vorhanden, die wechselweise beaufschlagt werden können, um während der Beaufschlagung des einen Flüssigkeitsabscheiders den anderen auf die gewünschte Abwassertemperatur abkühlen zu können. Den gleichen Zweck erfüllt ein einziger, entsprechend groß bemessener Flüssigkeitsabscheider

Diese Flüssigkeitsabscheider sind vorzugsweise als Zyklone ausgebildet, um eine möglichst vollständige Flüssigkeitsabscheidung zu erreichen. Ansonsten würde durch die im weiteren Verlauf angeordneten Vakuumpumpen ein Teil des Kondensats wieder verdampft und über die feuchtigkeitsempfindlichen Vakuumpumpen zusätzlich dem Gaswäscher zugeführt, was zu einer starken Vermehrung und Verdünnung der Waschflüssigkeit führen würde. Die im Gaswäscher im wesentlichen im Kreislauf geführte Umlaufflüssigkeit wird bei Erreichen der maximalen Schmutzbefrachtung ganz oder teilweise ebenfalls der Abwasserabführleitung der Kraftstation zugeführt.

Eine den Autoklaven umgehende Bypass-Leitung mit Prozeßdampf mündet in der Abführleitung zwischen dem Vorfilter und dem Sterilfilter, um die vorher beschriebene Reinigungsmöglichkeit für diese Filter zu bieten. Neben dem relativ hoch im Autoklaven-Innenraum angeordneten Absaug-Anschluß kann der Autoklav zusätzlich einen am tiefsten Punkt des Innenraums angeordneten Absauganschluß aufweisen, der vorzugsweise in der vorhandenen Absaugleitung vor dem Kondensator, aber nach den Filtern mündet, mittels einer Abzweigung jedoch auch umschaltbar auf eine Mündung vor dem Kondensator und den Filtern ist.

Die Kraftstation enthält ferner eine zwischen dem Kondensat-Sammelbehälter und der Prozeßdampfzuführung angeordnete Pumpe und entsprechende Rückführleitung, mit deren Hilfe das in den Zuführleitungen des Prozeßdampfes sowie im Mantelraum angefallene Kondensat wieder dem Prozeßdampf zur Sättigung zugeführt wird, bei nicht ausreichender Menge ggfs. unter Zusatz von Frischwasser.

Die Anschlüsse zwischen Autoklav und Kraftstation sind vorzugsweise als Kugelventile ausgebildet, da diese Bauform am besten eine Desinfektion der Kontaktflächen der Ventile durch die Überdruck-Dampfphase im Autoklaven ermöglicht.

Eine Ausführungsform gemäß der Erfindung ist nachfolgend anhand der Figuren beispielhaft näher beschrieben.

Es zeigen:
- Fig. 1: eine Prinzipdarstellung des an die Kraftstation angeschlossenen Autoklaven und
- Fig. 2: eine Querschnittsdarstellung des Gaswäschers.

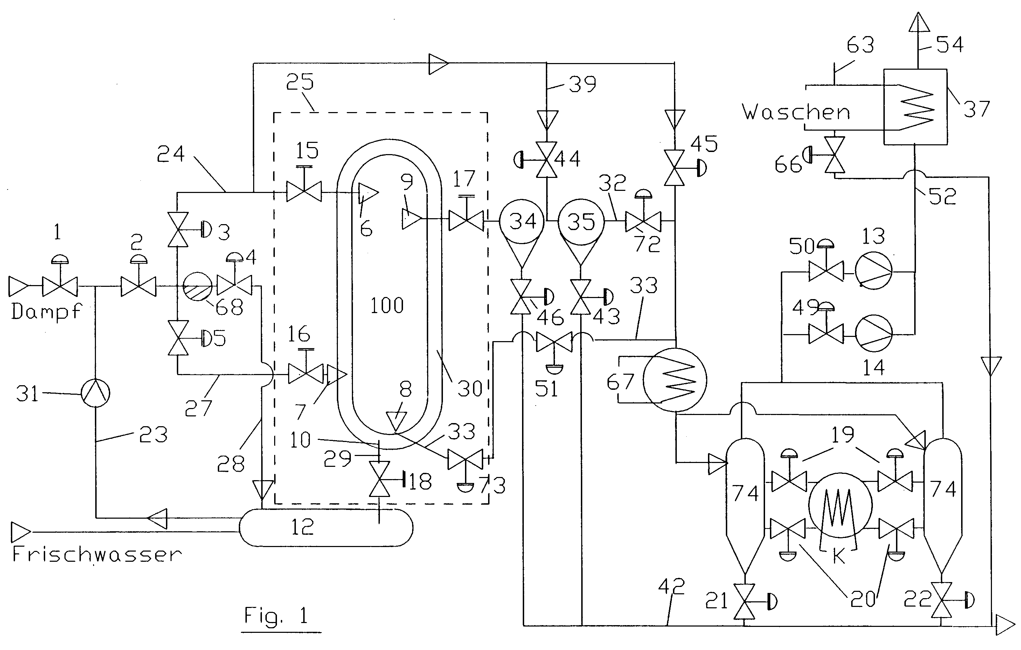

Fig. 1 zeigt den Autoklaven 25, dessen Bestandteile von einer gestrichelten Linie umfaßt sind:

Der Innenraum 100 des Autoklaven wird von einem doppelwandigen Mantel umgeben, wodurch der umgebende Mantelraum 30 gebildet wird. Im Innenraum mündet der Dampfanschluß 6 zum Eindringen des Prozeßdampfes sowie ein hochliegender Absauganschluß 9 zum Absaugen der Innenraum-Atmosphäre sowie ein tiefliegender Absauganschluß 8 am tiefsten Punkt des Autoklaven-Innenraumes, von dem aus das dort gesammelte Kondensat abgesaugt werden kann.

Die Zuführleitung 24 zum Dampfanschluß 6 sowie die Absaugleitungen 32 und 33, die von den Absauganschlüssen 8 und 9 des Innenraumes wegführen, sind jeweils durch Kupplungsventile 15, 17 und 73 absperrbar und von der Umgebung abkoppelbar, die damit die Schnittstelle des Autoklaven zur Umgebung darstellen.

Ebenso ist auch die im Dampfanschluß 7 des Mantelraumes 30 mündende Zuführleitung 27 von einem solchen Kupplungsventil 16 verschließbar und trennbar ausgebildet, sowie der vom tiefsten Punkt des Mantelraumes 30 wegführende Kondensat-Anschluß 10, dessen Abführleitung 29 ebenfalls ein solches Kupplungsventil 18 aufweist.

Alle außerhalb der gestrichelten Linie und damit des fahrbaren Autoklaven 25 dargestellten Elemente befinden sich in einer als Kraftstation bezeichneten Einheit, die in einem mobilen Container untergebracht ist und damit zwar weniger oft als der Autoklav selbst an einen anderen Standort gebracht wird, da dies die teilweise sehr empfindlichen Aggregate auf Dauer schädigen würde, aber dennoch in größeren zeitlichen Abständen einen Standortwechsel zuläßt.

Diese Kraftstation muß ihrerseits wieder mit Prozeßdampf zum Beaufschlagen des Autoklaven versorgt werden - wozu vorzugsweise an geeigneten Standorten Überschußkapazitäten etc. verwendet werden - sowie mit elektrischem Strom zur Versorgung der Steuerungseinheit und der Vielzahl von angesteuerten Ventilen sowie mit Frischwasser zur Sättigung des Prozeßdampfes und zum Verdünnen der Waschflüssigkeit des Gaswäschers 37.

Die Rückstände aus der Evakuierung des Autoklaven verlassen die Kraftstation in Gasform über die Auslaßleitung 54 des Gaswäschers 37 sowie in wässeriger Form über die Abwasserleitung 42, die in das örtlich vorhandene Abwassernetz mündet. Die Zuführung von Prozeßdampf in die Kraftstation wird über die Ventile 1 und 2 geregelt. Die gemäß dem fraktionierten Vakuum-Dampfverfahren notwendige intermittierende Einleitung von Prozeßdampf über die Zuführleitung 24 in den Innenraum 10 wird durch ein weiteres Ventil 3 geregelt, während Ventil 5 die Einleitung von Prozeßdampf über die Zuführleitung 27 in den Mantelraum 30 steuert, der üblicherweise annähernd durchgängig beheizt wird, um ein zu starkes Kondensieren des in den Innenraum 10 eingeleiteten Prozeßdampfes an der kühlen Außenfläche des Autoklaven zu vermeiden.

Das in den Zuführleitungen anfallende Kondensat aus Prozeßdampf wird in einem Kondensatsammler 68 aufgefangen und kann von dort mittels eines Ventils 4 in den zentralen Kondensat-Sammelbehälter 12 über eine Leitung 28 abgelassen werden. In den Kondensat-Sammelbehälter 12 wird auch das im Mantelraum 30 angesammelte Prozeßdampf-Kondensat vom Kondensat-Anschluß 10 aus über das Kupplungsventil 18 nach Bedarf abgelassen.

Der Inhalt des Kondensat-Sammlers 12, der ggfs. über einen Frischwasser-Anschluß aufgestockt werden kann, wird über eine Rückführleitung 23 und eine Pumpe 31 der Prozeßdampf-Zuführung zwischen den Ventilen 1 und 2 zur Sättigung des Prozeßdampfes mit Wasser wieder zugesetzt.

Die mit Prozeßdampf beaufschlagte Atmosphäre des Innenraumes 100 des Autoklaven wird nach jedem Zyklus über die Absaug-Anschlüsse 8 bzw. 9 abgesaugt und - getrennt nach flüssigen und gasförmigen Anteilen sowie spezifischer Nachbehandlung - der Umgebung wieder zugeführt.

Um sicherzustellen, daß die der Umgebung zugeführten Stoffe aus dem Autoklaven keimfrei sind, sind in der vom hochliegenden Absaug-Anschluß 9 wegführenden Absaugleitung 32 nach dem Kupplungsventil 17 ein Vorfilter 34 sowie ein Sterilfilter 35 angeordnet um im weiteren Verlauf ein Absperrventil 72. Auch eine direkt zwischen den Filtern 34 und 35 mündende Leitung 39, über die sauberer Prozeßdampf zugeführt werden kann, ist über ein Ventil 44 absperrbar. Gleiches gilt auch für die Abführleitungen, über die der Sumpf der Filter 34 und 35 mit der Abwasserleitung 42 verbunden ist. Auch diese sind über Ventile 42, 43 dicht absperrbar.

Bei Beaufschlagung des Innenraumes 100 des Autoklaven mit Prozeßdampf werden diese beiden Filter mitbeaufschlagt, indem das zwischen den Filtern und dem Autoklaven angeordnete Kupplungsventil 17 geöffnet bleibt, die die Filter umgebenden Ventile 42, 43, 44 und 72 jedoch geschlossen bleiben. Obwohl diese Filter in der Kraftstation angeordnet sind, sind sie Teil des mit Dampf beaufschlagten Bereiches. Dadurch werden die in den Filtern 34, 35 zurückgehaltenen Stoffe desinfiziert. Die sich als Flüssigkeit im Filtersumpf ansammelnden Reststoffe können damit bei Bedarf nach erfolgter Desinfektion über die Ventile 42, 43 problemlos an die Abwasserleitung 42 abgegeben werden.

Der Vorfilter 34 besitzt eine Durchlaßgröße von 1,2 µm und dient dem Zurückhalten von solchen Feststoffen und Flüssigkeitströpfchen, die den nachfolgenden Sterilfilter 35 sofort verstopfen würden.

Der Vorfilter 34 kann über in Gegenrichtung zugeführten Prozeßdampf aus der Leitung 39, die zwischen Vorfilter und Sterilfilter 35 mündet, rückgespült werden.

Bei geschlossenem Kupplungsventil 17 werden die im Vorfilter 34 befindlichen Stoffe dabei in den Sumpf des Vorfilters 34 gespült und gelangen somit in die Abwasserleitung 42. Im Gegensatz zum Vorfilter 34 kann der Sterilfilter 35 nach dem Zusetzen nicht wieder regeneriert werden. Durch Beaufschlagen über die Leitung 39 mit Prozeßdampf bei geschlossenen umgebenden Ventilen kann der Filterkuchen des nur 0,2 µm feinen Sterilfilters 35, der auch Keime wie Bakterien und Viren zurückhält, jedoch mit ausreichend langer Haltezeit und ausreichendem Druck zur Sicherheit - ebenso wie der Vorfilter 34 und dessen Filtersumpf - bis zum Erreichen einer Desinfektion bzw. Sterilisation beaufschlagt werden, bevor der Filterkuchen des Sterilfilters 35 entsorgt und der Filtersumpf beider Filter jeweils in die Abwasserleitung 42 abgelassen wird.

Die aus dem Innenraum 100 mittels parallel geschalteter Vakuumpumpen 13, 14 abgesaugte Atmosphäre wird im weiteren Verlauf über eine Leitung 40 einem Kondensator 67 zugeführt, um ein weitestgehendes Kondensieren der in den Abluftbrüden enthaltenen Dämpfe zu erzielen. Anschließend wird das dabei gebildete Kondensat sowie die restlichen gasförmigen Bestandteile wechselweise einem von zwei parallelen FLüssigkeitsabscheidern in Form von Zyklonen 74 auf möglichst kurzem Leitungswege zugeführt, in denen der Flüssiganteil abgeschieden und über Ventile 21, 22 der Abwasserleitung 42 zugeführt wird. Diese Flüssigkeitsabscheidung mittels Zyklon ist notwendig, damit durch die Saugwirkung der nach den Flüssigkeitsabscheidern 74 und dem Kondensator 67 angeordneten Vakuumpumpen 13, 14 nicht ein erheblicher Teil des gebildeten Kondensats wieder verdampft wird.

Bei der Flüssigkeitsabscheidung ist darauf zu achten, daß die Einleitung des Kondensates in die Abwasserleitung 42 mit einer Temperatur annähernd der Umgebungstemperatur , also etwa 20°C, geschieht. Da das im Kondensator 67 anfallende Kondensat in der Regel wesentlich wärmer ist, werden die beiden Flüssigkeitsabscheider 74 wechselweise beschickt, wobei der Inhalt des momentan nicht beschickten Flüssigkeitsabscheiders 74 mit Hilfe eines Kühlers K und entsprechender Ventile 19, 20 heruntergekühlt wird. Um für die Kraftstation nicht zusätzlich zum Frischwasser noch größere Mengen Kühlwasser zu benötigen, ist der Kühler K mit Umgebungsluft gekühlt.

Die in den Zyklonen 74 abgeschiedenen gasförmigen Anteile werden über die - mittels der Ventile 49, 50 einzeln zu- und abschaltbaren - Vakuumpumpen 13, 14 abgesaugt und über einen Gaswäscher 37 an die Umgebungsluft abgegeben.

Neben der beschriebenen Absaugung der Atmosphäre über einen hochliegenden Absauganschluß 9 befindet sich im Innenraum 100 des Autoklaven ein tiefliegender Absauganschluß 8 am tiefsten Punkt des Innenraumes 100. Die Absaugleitung 33 von diesem tiefliegenden Absauganschluß mündet in der Absaugleitung 32 unmittelbar vor dem Kondensator 67. Wenn von diesem tiefliegenden Anschluß 8 aus zunächst das Kondensat im Innenraum 10 abgepumpt wird, so durchläuft es den Kondensator 67 nutzlos.

Die aus demselben Anschluß 8 anschließend abgesaugten, im wesentlichen gasförmigen, Brüden müssen dagegen diesen Kondensator 67 zur Kondensierung durchlaufen.

Ein Durchlaufen der Filter der von diesem tiefliegenden Anschluß 8 abgesaugten Brüden ist nicht notwendig, da von dem tiefliegenden Anschluß 8 nur nach der abschließenden Überdruck-Dampfphase abgesaugt wird, die zu einer Desinfizierung oder gar Sterilisierung des Inhaltes des Autoklaven führt.

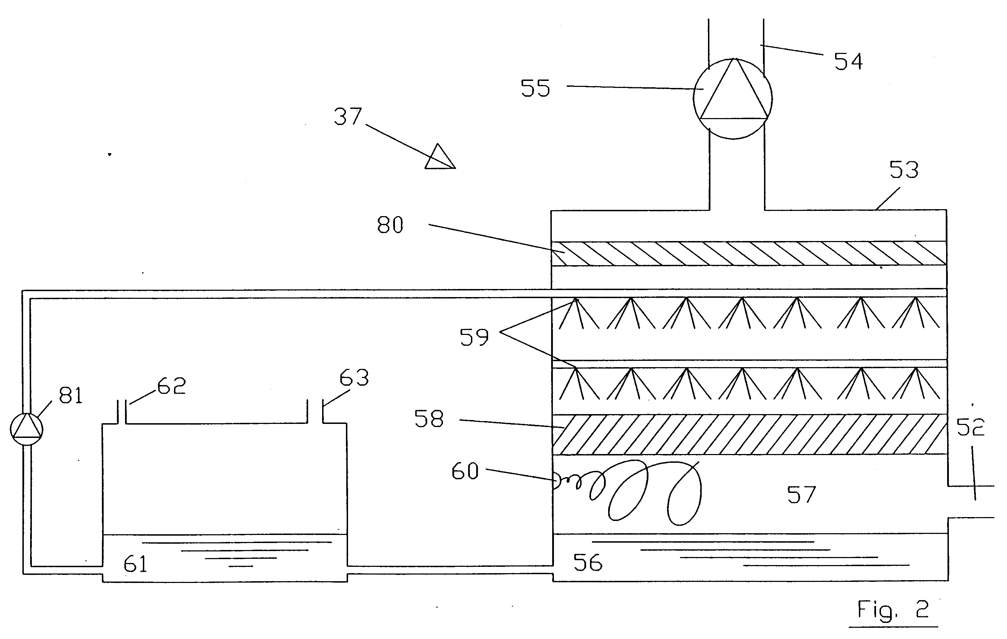

Im Gaswäscher 37, der im einzelnen in Fig. 2 dargestellt ist, läuft eine Umlaufflüssigkeit im wesentlichen im Kreis um, die mit zunehmender Verschmutzung zusätzlich mittels Frischwasser verdünnt werden kann und ganz oder teilweise über das Ventil 66 in die Abwasserleitung 42 abgelassen werden kann.

Wie Fig. 2 im einzelnen zeigt, besteht der Gaswäscher 37 aus einem Gehäuse 53 in dem die zu reinigenden Gase im wesentlichen von unten nach oben hindurchgeführt werden und dabei verschiedene Schichten durchlaufen, in denen die die Geruchsstoffe tragenden Bestandteile aus dem Gas mittels derr Umlaufflüssigkeit, die im wesentlichen im Gegenstrom geführt wird, herausgewaschen werden.

Zu diesem Zweck befindet sich auf dem Grund des Gehäuses 53 eine interne Vorlage 56 aus Umlaufflüssigkeit. Über deren Flüssigkeitsspiegel wird das zu reinigende Gas über die Leitung 52 in das Gehäuse eingebracht und steigt aufgrund einer in der Auslaßleitung 54 nach dem Gawäscher 37 angeordneten Saugpumpe 55 nach oben. Oberhalb der internen Vorlage 56 befindet sich ein Nebelbereich 57, in dem mittels Ultraschall-Nebeldüsen 60 ein Feinstnebel aus nicht verunreinigter Waschflüssigkeit erzeugt wird, die anschließend als Umlaufflüssigkeit benutzt wird. An diesen Nebeltröpfchen lagern sich die Geruchsstoffe der zu reinigenden Gase an.

Darüber befindet sich eine Abscheideschicht 58 aus einem Feststoff mit hoher spezifischer Oberfläche wie etwas Lava, die darüber hinaus ausreichend gasdurchlässig sein muß. Diese Abscheideschicht 58 wird zusätzlich von oben mittels Sprinklern 59 mit Umlaufflüssigkeit besprüht, um das Auswaschen der Geruchsstoffe aus den Gasen zu verbessern. Oberhalb der Sprinkler 59 befindet sich ein an sich bekannter Tropfenabscheider 80, der nicht von Umlaufflüssigkeit befeuchtet wird und der Rückhaltung möglichst aller Flüssigkeitströpfchen dient.

Die in Sprinklern 59 über eine Leitung 65 zugeführte Umlaufflüssigkeit ist einer externen Vorlage 61 der Waschflüssigkeit entnommen, die als Zwischenspeicher dient und mittels einer Pumpe 81 zu den Sprinklern 59 geführt wird. In der externen Vorlage 61 ist neben einem Frischwasser-Anschluß 63 zum Verdünnen der Umlaufflüssigkeit auch ein Ablaßventil 66 zur Abwasserleitung 42 hin angeordnet, sowie ein Belüftungsanschluß 62.

Die diesen Kontaktwäscher verlassenden Gase sind soweit von geruchsintensiven Inhaltsstoffen befreit, daß in der Umgebung der Kraftstation keine Geruchsbelästigung eintritt.

## Patentansprüche

1. Verfahren zum Desinfizieren von Abfällen und dgl. nach dem fraktionierten Vakuum-Dampfverfahren mit mehreren Normaldruck-Dampfphasen, anschließender Überdruck-Dampfphase und jeweils folgender Evakuierung in Autoklaven, insbesondere transportablen Autoklaven,
**dadurch gekennzeichnet, daß**
a) beim Evakuieren nach den Normaldruck-Dampfphasen die Atmosphäre von einem hochliegenden Absaugeanschluß des Autoklaven aus abgesaugt und
- von Keimen gereinigt wird,
- der Dampfanteil kondensiert und als Flüssiganteil mit annähernd Umgebungstemperatur abgeschieden wird,
- die Gasanteile von Geruchsstoffen befreit und an die Umgebung abgeführt werden,
b) beim Evakuieren nach der Überdruck-Dampfphase
- vom etwa tiefsten Punkt des Autoklaven aus zunächst das dort angesammelte Kondensat entfernt und mit annähernd Umgebungstemperatur dem Abwasser zugeführt wird, und
- anschließend von etwa dem gleichen Punkt des Autoklaven aus die Atmosphäre abgesaugt und vor der Abgabe an die Umgebung von Geruchsstoffen befreit wird,
c) wobei die Einrichtung zum Entfernen der Keime beim Beaufschlagen des Autoklaven mit Prozeßdampf mit dem Autoklaven verbunden ist und den gleichen Verhältnissen unterworfen werden.

2. Verfahren nach Anspruch 1,
**dadurch gekennzeichnet, daß**
beim Evakuieren nach der Überdruck-Dampfphase nach dem Absaugen des Kondensates die abgesaugten Brüden vor der Reinigung von Geruchsstoffen kondensiert werden.

3. Verfahren zum Desinfizieren von Abfällen und dgl. nach dem fraktionierten Vakuum-Dampfverfahren mit mehreren Normaldruck-Dampfphasen, anschließender Überdruck-Dampfphase und jeweils folgender Evakuierung in Autoklaven, insbesondere transportablen Autoklaven,
**dadurch gekennzeichnet, daß**
a) beim Evakuieren nach den Normaldruck-Dampfphasen die Atmosphäre von einem hochliegenden Absaugeanschluß des Autoklaven aus abgesaugt und
- von Keimen gereinigt wird,
- die Dampfanteile kondensiert und als Flüssiganteil mit annähernd Umgebungstemperatur abgeschieden werden,
- die Gasanteile von Geruchsstoffen befreit und an die Umgebung abgeführt werden,
b) beim Evakuieren nach der Überdruck-Dampfphase
- vom etwa tiefsten Punkt des Autoklaven aus zunächst das dort angesammelte Kondensat entfernt und mit annähernd Umgebungstemperatur dem Abwasser zugeführt wird,
- nach dem Absaugen des Kondensates die Atmosphäre von einem relativ hohen Punkt des Autoklaven aus abgesaugt und
- die Dampfanteile der Atmosphäre kondensiert und als Flüssiganteil mit annähernd Umgebungstemperatur ins Abwasser abgeschieden werden und
- die Gasanteile nach Entfernen der Geruchssstoffe an die Umgebung abgeführt werden
c) wobei die Einrichtung zum Entfernen der Keime beim Beaufschlagen des Autoklaven mit Prozeßdampf mit dem Autoklaven verbunden sind und den gleichen Verhältnissen unterworfen werden.

4. Verfahren nach einem der vorhergehenden Ansprüche,
**dadurch gekennzeichnet, daß**
beim Evakuieren nach der Überdruck-Dampfphase die Atmosphäre von Feststoffen gereinigt wird.

5. Verfahren nach einem der vorhergehenden Ansprüche,
**dadurch gekennzeichnet, daß**
der Autoklav mit Prozeßdampf nicht nur beaufschlagt, sondern zusätzlich von außen beheizt wird.

6. Verfahren nach einem der vorhergehenden Ansprüche,
**dadurch gekennzeichnet, daß**
der Prozeßdampf mit Flüssigkeit gesättigt wird, indem das in den Zuführleitungen und der Beheizung des Autoklaven anfallende Kondensat, ggfs. ergänzt um Frischwasser, dem Prozeßdampf wieder zugesetzt wird.

7. Verfahren nach einem der vorhergehenden Ansprüche,
**dadurch gekennzeichnet, daß**
die Reinigung der abgesaugten Atmosphäre des Autoklaven von Keimen und/oder Feststoffen mittels wenigstens zweier Filter mit abnehmender Durchgangsgröße geschieht, von denen
- wenigstens der gröbste Filter gegen die normale Durchlaufrichtung mittels Prozeßdampf gespült werden kann und
- wenigstens der feinste der Filter in der normalen Durchlaufrichtung mit Prozeßdampf beaufschlagt werden kann.

8. Verfahren nach einem der vorhergehenden Ansprüche,
**dadurch gekennzeichnet, daß**
die Entfernung der Geruchsstoffe erfolgt, indem
- die Gase durch eine Nebelzone geführt werden, deren Nebel aus feinsten Flüssigkeitströpfchen einer unverschmutzten Waschflüssigkeit bestehen, wie sie z.B. mittels der an sich bekannten Ultraschall-Nebeldüsen erzeugt werden können,
- die Gase anschließend durch eine Abscheideschicht aus einem Feststoff mit großer Oberfläche wie etwa Lava oder Tuffstein im Gegenstrom zu einer Umlaufflüssigkeit geführt werden, und
- die Gase anschließend über einen an sich bekannten Tropfenabscheider in die Umgebung abgesaugt werden,
- wobei die Umlaufflüssigkeit im Kreis geführt wird und Wasser als Verdünnungsmedium der Waschflüssigkeit dient.

9. Verfahren nach Anspruch 7,
**dadurch gekennzeichnet, daß**
die Gase im Saugbetrieb durch die Nebelzone und die Abscheideschicht geführt werden.

10. Verfahren nach einem der vorhergehenden Ansprüche,
**dadurch gekennzeichnet, daß**
die Entfernung der Geruchsstoffe erfolgt, indem die Ablüftbrüden über einen Adsorptionsfilter geführt werden, der Aktivkohle enthält.

11. Vorrichtung zum Desinfizieren von Abfällen, insbesondere nach einem der Ansprüche 1 bis 10,
**gekennzeichnet durch**
a) einen mobilen, doppelwandigen Autoklaven (25) mit Prozeßdampf-Anschlüssen (6, 7) zum Innenraum (100) und zum Mantelraum (30) sowie wenigstens einem an einem relativ hohen Punkt des Autoklaven angeordneten Innenraum-Absauganschluß (9) und einen am tiefsten Punkt des Mantelraumes angeordneten Mantelraum-Kondensat-Ableitungsanschluß (10), und
b) eine Kraftstation mit
- einer Steuereinheit,
- wenigstens zwei in Reihe geschalteten Feststoff-Filtern (34, 35)in der Innenraum-Absaugleitung (32),
- wenigstens einem Kondensator (67) für die abgesaugte Atmosphäre des Innenraumes (100),
- wenigstens einem Flüssigkeits-Abscheider,
- wenigstens einer Vakuum-Pumpe (13, 14),
- wenigstens einer Anlage zur Abluft-Desodorierung und
- einer Abwasserleitung (42), die die Flüssigkeit aus den Filtern (34, 35) und den Flüssigkeitsabscheidern in das Abwasser abführt.

12. Vorrichtung nach Anspruch 11,
**dadurch gekennzeichnet, daß**
zwei wahlweise ansteuerbare Flüssigkeits-Abscheider vorhanden sind.

13. Vorrichtung nach Anspruch 11 oder 12,
**dadurch gekennzeichnet, daß**
vier parallel geschaltete Vakuum-Pumpen vorhanden sind.

14. Vorrichtung nach einem der Ansprüche 11 bis 13,
**dadurch gekennzeichnet, daß**
die Anlage zur Abluft-Desodorierung ein Gaswäscher ist.

15. Vorrichtung nach einem der Ansprüche 11 bis 14,
**dadurch gekennzeichnet, daß**
die Flüssigkeitsabscheider sehr große Flüssigkeitsbehälter aufweisen.

16. Vorrichtung nach einem der Ansprüche 11 bis 15,
**dadurch gekennzeichnet, daß**
der Autoklav (25) zusätzlich einen Innenraum-Absauganschluß (8) am tiefsten Punkt des Innenraumes (100) aufweist.

17. Vorrichtung nach einem der Ansprüche 11 bis 16,
**gekennzeichnet durch**
einen Kondensat-Sammelbehälter (12) für das Kondensat aus den Prozeßdampf-Zuführleitungen (24, 27) und dem Mantelraum (30) mit einer Rückführleitung (23) und einer Pumpe (31) zum Rückführen des Kondensates in die Prozeßdampf-Zufuhr, sowie mit einem Frischwasser-Anschluß.

18. Vorrichtung nach einem der Ansprüche 11 bis 17,
**dadurch gekennzeichnet, daß**
der Kondensator (67) luftgekühlt ist.

19. Vorrichtung nach einem der Ansprüche 11 bis 18,
**dadurch gekennzeichnet, daß**
die Flüssigkeitsabscheider als Zyklone (74) ausgebildet sind.

20. Vorrichtung nach einem der Ansprüche 11 bis 19,
**dadurch gekennzeichnet, daß**
die FLüssigkeitsabscheider luftgekühlt sind.

21. Vorrichtung nach einem der Ansprüche 11 bis 20,
**dadurch gekennzeichnet, daß**
zur Keimabscheidung und/oder FeststoffAbscheidung zwei Filter (34, 35) in Reihe angeordnet sind, von denen der Vorfilter (34) eine Durchlaßgröße von etwa 1,2 µm und der anschließende Sterilfilter (35) eine Durchlaßgröße von etwa 0,2 µm besitzt.

22. Vorrichtung nach Anspruch 21,
**dadurch gekennzeichnet, daß**
eine Prozeßdampf-Zuführung (39) direkt zwischen dem Vorfilter (34) und dem Sterilfilter (35) mündet.

23. Vorrichtung nach einem der Ansprüche 11 bis 22,
**dadurch gekennzeichnet, daß**
die Leitung (33) vom tiefsten Punkt des Innenraumes (100) in der Absaugleitung (32) des hochliegenden Innenraum-Anschlusses (9) nach den Filtern (34, 35) und vor dem Kondensator (67) mündet.

24. Vorrichtung nach einem der Ansprüche 11 bis 23,
**dadurch gekennzeichnet, daß**
der Gaswäscher aus im wesentlichen vertikal übereinander angeordneten Bereichen besteht, durch welche die Gase von unten nach oben hindurchgeführt werden, wobei in Durchlaufrichtung nach dem Gaswäscher eine Saugpumpe angeordnet ist und durch den Gaswäscher im wesentlichen im Gegenstrom, also von oben nach unten, eine Umlaufflüssigkeit im Kreis geführt wird, wobei der Gaswäscher
- im untersten Bereich in einer Wanne eine Ansammlung von Umlaufflüssigkeit aufweist,
- in dem darüberliegenden Bereich das zu reinigende Gas eingebracht und mittels Ultraschall-Nebeldüsen aus unverschmutzter Waschflüssigkeit ein Feinstnebel erzeugt wird,
- in einer darüber angeordneten Abscheideschicht aus einem Feststoff mit hoher Oberfläche wie Lavagestein, Tuffstein oder ähnlichem, die von oben mittels einer Sprüheinrichtung mit Umlaufflüssigkeit besprüht wird, die Luftinhaltstoffe ausgewaschen werden, und
- in einer über der Sprüheinrichtung angeordneten Tropfenabscheideschicht die Flüssigkeitsanteile der hindurchtretenden, abgeführten Gase zurückgehalten werden, wobei
- die in der Sprüheinrichtung verwendete Umlaufflüssigkeit der Ansammlung an Umlaufflüssigkeit am Boden des Wäschers entnommen wird.

25. Vorrichtung nach Anspruch 24,
**dadurch gekennzeichnet, daß**
im Kreislauf der Waschflüssigkeit außerhalb des Gaswäschers ein externer Zwischenspeicher für Waschflüssigkeit angeordnet ist, der eine Belüftungsöffnung sowie einen Frischwasserzulauf aufweist.

26. Vorrichtung nach einem der Ansprüche 11 bis 25,
**dadurch gekennzeichnet, daß**
die Vakuumpumpen eine Heizeinrichtung aufweisen.

27. Vorrichtung nach einem der Ansprüche 11 bis 26,
**dadurch gekennzeichnet, daß**
die Kraftstation innerhalb eines mobilen Containers untergebracht ist.

## Claims

1. A process for disinfecting waste and the like using the fractional vacuum vapour method with a plurality of normal pressure vapour phases, a subsequent increased pressure vapour phase and respectively following evacuation in autoclaves, in particular transportable autoclaves,
characterised in that
a) in the evacuation operation after the normal pressure vapour phases the atmosphere is sucked out of a high-level suction removal connection of the autoclave and
- cleansed of germs,
- the vapour component is condensed and separated as a liquid component at approximately ambient temperature,
- the gas components are freed of odorous substances and discharged to the environment,
b) in the evacuation operation after the increased pressure vapour phase
- the condensate which has accumulated at the approximately lowest point of the autoclave is firstly removed from the approximately lowest point of the autoclave and fed at approximately ambient temperature to the waste water, and
- then the atmosphere is sucked away from approximately the same point of the autoclave and freed of odorous substances before discharge to the environment, and
c) when the autoclave is acted upon by process vapour the device for the removal of the germs is connected to the autoclave and is subjected to the same conditions.

2. A process according to claim 1 characterised in that in the evacuation operation after the increased pressure vapour phase after suction removal of the condensate the sucked-away vapours are condensed prior to the operation of removing odorous substances.

3. A process for disinfecting waste and the like using the fractional vacuum vapour method with a plurality of normal pressure vapour phases, a subsequent increased pressure vapour phase and respectively following evacuation in autoclaves, in particular transportable autoclaves,
characterised in that
a) in the evacuation operation after the normal pressure vapour phases the atmosphere is sucked out of a high-level suction removal connection of the autoclave and
- cleansed of germs,
- the vapour components are condensed and separated as a liquid component at approximately ambient temperature,
- the gas components are freed of odorous substances and discharged to the environment,
b) in the evacuation operation after the increased pressure vapour phase
- the condensate which has accumulated at the approximately lowest point of the autoclave is firstly removed from the approximately lowest point of the autoclave and fed at approximately ambient temperature to the waste water,
- after the operation of sucking away the condensate the atmosphere is sucked away from a relatively high point of the autoclave, and
- the vapour components of the atmosphere are condensed and separated in the form of a liquid component at approximately ambient temperature into the waste water, and
- the gas components after removal of the odorous substances are discharged to the environment, and
c) when the autoclave is acted upon by process vapour the device for the removal of the germs is connected to the autoclave and is subjected to the same conditions.

4. A process according to one of the preceding claims characterised in that in the evacuation operation after the increased pressure vapour phase the atmosphere is cleansed of solid materials.

5. A process according to one of the preceding claims characterised in that the autoclave is not only acted upon by process vapour but it is additionally heated from the exterior.

6. A process according to one of the preceding claims characterised in that the process vapour is saturated with liquid by the condensate which occurs in the feed lines and the heating means of the autoclave, possibly supplemented by fresh water, being added to the process vapour again.

7. A process according to one of the preceding claims characterised in that the operation of cleansing the sucked-away atmosphere of the autoclave of germs and/or solid materials is effected by means of at least two filters with decreasing passage sizes of which
- at least the coarsest filter can be flushed by means of process vapour in opposite relationship to the normal through-flow direction, and
- at least the finest of the filters can be acted upon by process vapour in the normal through-flow direction.

8. A process according to one of the preceding claims characterised in that the operation of removing the odorous substances is effected by a procedure whereby
- the gases are passed through a mist zone whose mists comprise very fine liquid droplets of an uncontaminated washing liquid as they can be produced for example by means of the per se known ultrasound atomising nozzles,
- the gases are then passed through a separation layer comprising a solid material with a large surface area such as for example lava or tuff rock in counter-flow to a circulating liquid, and
- the gases are then sucked away into the environment by way of a per se known drop separator,
- wherein the circulating liquid is circulated and water serves as a diluent for the washing liquid.

9. A process according to claim 7 characterised in that the gases are passed in a suction mode through the mist zone and the separation layer.

10. A process according to one of the preceding claims characterised in that the operation of removing the odorous substances is effected by the exhaust air vapours being passed over an adsorption filter which includes activated carbon.

11. Apparatus for disinfecting waste, in particular according to one of claims 1 to 10, characterised by
a) a mobile, double-walled autoclave (25) having process vapour connections (6, 7) to the interior space (100) and to the jacket space (30) and at least one interior space suction removal connection (9) arranged at a relatively high point of the autoclave and a jacket space condensate drain connection (10) arranged at the lowest point of the jacket space, and
b) a power station comprising
- a control unit,
- at least two series-connected solid material filters (34, 35) in the interior space suction removal conduit (32),
- at least one condenser (67) for the sucked-away atmosphere of the interior space (100),
- at least one liquid separator,
- at least one vacuum pump (13, 14),
- at least one installation for exhaust air deodorising, and
- a waste water conduit (42) which discharges the liquid from the filters (34, 35) and the liquid separators into the waste water.

12. Apparatus according to claim 11 characterised in that there are two selectively actuable liquid separators.

13. Apparatus according to claim 11 or claim 12 characterised in that there are four parallel-connected vacuum pumps.

14. Apparatus according to one of claims 11 to 13 characterised in that the exhaust air deodorisation installation is a gas washer.

15. Apparatus according to one of claims 11 to 14 characterised in that the liquid separators have very large liquid containers.

16. Apparatus according to one of claims 11 to 15 characterised in that the autoclave (25) additionally has an interior space suction removal connection (8) at the lowest point of the interior space (100).

17. Apparatus according to one of claims 11 to 16 characterised by a condensate collecting container (12) for the condensate from the process vapour feed lines (24, 27) and the jacket space (30) with a return line (23) and a pump (31) for return of the condensate into the process vapour feed, and with a fresh water connection.

18. Apparatus according to one of claims 11 to 17 characterised in that the condenser (67) is air-cooled.

19. Apparatus according to one of claims 11 to 18 characterised in that the liquid separators are in the form of cyclone separators (74).

20. Apparatus according to one of claims 11 to 19 characterised in that the liquid separators are air-cooled.

21. Apparatus according to one of claims 11 to 20 characterised in that for germ separation and/or solid material separation there are two filters (34, 35) arranged in series, of which the first filter (34) has a passage size of about 1.2 µm and the subsequent sterile filter (35) has a passage size of about 0.2 µm.

22. Apparatus according to claim 21 characterised in that a process vapour feed (39) opens directly between the first filter (34) and the sterile filter (35).

23. Apparatus according to one of claims 11 to 22 characterised in that the line (33) from the lowest point of the interior space (100) communicates with the suction removal line (32) of the high-level interior space connection (9) downstream of the filters (34, 35) and upstream of the condenser (67).

24. Apparatus according to one of claims 11 to 23 characterised in that the gas washer comprises regions which are arranged in substantially vertically superposed relationship and through which the gases are passed from below upwardly, wherein arranged downstream of the gas washer in the through-flow direction is a suction pump and a circulating liquid is circulated through the gas washer in substantially counter-flow relationship, that is to say from above downwardly, wherein
- the gas washer has an accumulation of circulating liquid in the lowermost region in a trough,
- in the region thereabove the gas to be cleaned is introduced and a very fine mist is produced from uncontaminated washing liquid by means of ultrasound atomising nozzles,
- in a separation layer arranged thereabove and comprising a solid material with a large surface area such as lava rock, tuff rock or the like which is sprayed with circulating liquid from above by means of a spray device, the airborne content substances are washed out, and
- in a drop separation layer arranged above the spray device the liquid components of the discharged gases passing therethrough are retained therein, wherein
- the circulating liquid used in the spray device is taken from the accumulation thereof at the bottom of the washer.

25. Apparatus according to claim 24 characterised in that arranged in the circuit of the washing liquid outside the gas washer is an external intermediate storage means for washing liquid, which has a vent opening and a fresh water feed means.

26. Apparatus according to one of claims 11 to 25 characterised in that the vacuum pumps have a heating means.

27. Apparatus according to one of claims 11 to 26 characterised in that the power station is disposed within a mobile container.

## Revendications

1. Procédé pour la désinfection de déchets et similaires selon le procédé de génération de vapeur sous vide, fractionné en plusieurs phases vapeur à la pression atmosphérique, suivies d'une phase vapeur en surpression ainsi que de l'obtention du vide en autoclave, en particulier dans des autoclaves transportables,
caractérisé
a) en ce qu'à l'obtention du vide après les phases vapeur à la pression atmosphérique, l'atmosphère est aspirée depuis un raccordement surélevé d'aspiration de l'autoclave et
- est purifiée des germes,
- la part vapeur est condensée et est évacuée sous forme liquide approximativement à température ambiante,
- les parts gazeuses sont débarrassées des substances odorantes et sont délivrées à l'environnement,
b) en ce qu'à l'obtention du vide après les phases vapeur en surpression,
- le condensat, qui s'est accumulé au point le plus bas de l'autoclave? est tout d'abord éliminé et est amené, approximativement à la température ambiante, aux eaux usées,
- puis l'atmosphère est aspirée approximativement depuis le même point de l'autoclave et est débarrassée des substances odorantes avant d'être délivrée à l'environnement,
c) le dispositif d'élimination des germes, relié à l'autoclave lorsque l'autoclave subit l'impact de la valeur de processus, étant soumis aux mêmes conditions.

2. Procédé selon la revendication 1,
caractérisé en ce que lors de l'obtention du vide après la phase vapeur en surpression et l'aspiration du condensat, les buées aspirées sont condensées avant d'être purifiées des substances odorantes.

3. Procédé pour la désinfection de déchets et similaires selon le procédé de génération de vapeur sous vide, fractionné en plusieurs phases vapeur à la pression atmosphérique, suivies d'une phase vapeur en surpression ainsi que de l'obtention du vide en autoclave, en particulier dans des autoclaves transportables,
caractérisé
a) en ce qu'à l'obtention du vide après les phases vapeur à la pression atmosphérique, l'atmosphère est aspirée depuis un raccordement surélevé d'aspiration de l'autoclave et
- est purifiée des germes,
- les parts vapeur sont condensées et évacuées sous forme liquide approximativement à température ambiante,
- les parts gazeuses sont débarrassées des substances odorantes et sont dégagées à l'environnement,
b) en ce qu'à l'obtention du vide après les phases vapeur en surpression,
- le condensat, qui s'est accumulé au point le plus bas de l'autoclave? est tout d'abord éliminé et est amené, approximativement à température ambiante, aux eaux usées,
- après l'aspiration du condensat, l'atmosphère est aspirée depuis un point relativement élevé de l'autoclave et
- les parts vapeur de l'atmosphère se condensent et sont évacuées dans les eaux usées sous forme liquide approximativement à température ambiante, et
- les parts gazeuses sont délivrées à l'atmosphère après élimination des substances odorantes,
c) le dispositif d'élimination des germes, relié à l'autoclave lorsque l'autoclave subit l'impact de la vapeur de processus, étant soumis aux mêmes conditions.

4. Procédé selon l'une des revendications précédentes,
caractérisé
en ce qu'à l'obtention du vide après la phase vapeur en surpression, l'atmosphère est purifiée des matières solides.

5. Procédé selon l'une des revendications précédentes,
caractérisé en ce que
l'autoclave subit non seulement la vapeur de processus mais est également chauffé en supplément de l'extérieur.

6. Procédé selon l'une des revendications précédentes,
caractérisé en ce que
la vapeur de processus est saturée avec du liquide, en réintégrant, à la vapeur de processus, le condensat en formation dans les conduites d'amenée et dans le chauffage de l'autoclave, complétée éventuellement par de l'eau fraîche.

7. Procédé selon l'une des revendications précédentes,
caractérisé en ce que
la purification des germes et/ou des matières solides présents dans l'atmosphère aspirée de l'autoclave s'effectue au moyen d'au moins deux filtres dont le passage est de dimension décroissante, parmi lesquels,
- au moins le filtre le plus grossier peut être nettoyé au moyen de la vapeur de processus dans le sens contraire à la direction normale de l'écoulement et
- au moins le filtre le plus fin peut subir l'impact de la vapeur de processus dans la direction normale de l'écoulement.

8. Procédé selon l'une des revendications précédentes,
caractérisé en ce que
l'élimination des substances odorantes s'effectue de la manière suivante
- les gaz sont dirigés à travers une zone de brouillard dont le brouillard se compose de très fines gouttelettes d'un liquide de lavage non souillé, tel que l'on peut obtenir par exemple à l'aide des buses à brouillard à ultrasons, connues en soi,
- les gaz sont ensuite amenéS à contre courant à un liquide de circulation en passant à travers une couche de séparation composée d'une matière solide de grande surface, telle que par exemple la roche volcanique ou le tuf, et
- les gaz sont finalement aspirés et rejetés à l'environnement par l'intermédiaire d'un pare-gouttes connu en soi,
- le liquide de circulation s'écoulant en circuit et l'eau servant de diluant au liquide de lavage.

9. Procédé selon la revendication 7,
caractérisé en ce que
les gaz sont guidés en mode d'aspiration à travers la zone de brouillard et la couche de séparation.

10. Procédé selon l'une des revendications précédentes,
caractérisé en ce que
l'élimination des substances odorantes s'effectue en dirigeant les buées de l'air d'échappement par un filtre d'adsorption contenant du charbon actif.

11. Dispositif pour la désinfection des déchets, en particulier selon l'une de revendications 1 à 10,
caractérisé par
a) un autoclave mobile à double paroi (25) présentant des raccordements (6, 7) de la vapeur de processus à l'espace intérieur (100) et à l'espace d'enveloppe (30) ainsi qu'au moins un raccordement d'aspiration (9) à l'espace intérieur, disposé à un point relativement élevé de l'autoclave et un raccordement (10) de conduite d'évacuation du condensat d'espace d'enveloppe, disposé au point le plus bas de l'espace d'enveloppe, et
b) par une station génératrice présentant
- une unité de commande,
- au moins deux filtres (34, 35) de matières solides, montés en série et se trouvant dans la conduite intérieure d'aspiration (32) de l'espace intérieur,
- au moins un condenseur (67) pour l'atmosphère aspirée de l'espace intérieur (100),
- au moins un séparateur de liquide,
- au moins une pompe à vide (13, 14),
- au moins une installation de neutralisation des mauvaises odeurs de l'air d'échappement et
- une conduite (42) des eaux usées, qui évacue, dans les eaux usées, le liquide provenant des filtres (34, 35) et des séparateurs de liquide.

12. Dispositif selon la revendication 11 ou 12,
caractérisé en ce qu'
il est prévu deux séparateurs de liquide, à commande sélective.

13. Dispositif selon la revendication 11 ou 12
caractérisé en ce qu'
il est prévu quatre pompes à vide montées en parallèle.

14. Dispositif selon l'une des revendications 11 à 13,
caractérisé en ce que
l'installation de désodorisation de l'air d'échappement est un laveur de gaz.

15. Dispositif selon l'une des revendications 11 à 14,
caractérisé en ce que les séparateurs de liquide présentent des récipients de liquide de très grande capacité.

16. Dispositif selon l'une des revendications 11 à 15,
caractérisé en ce que
l'autoclave (25) présente en supplément un raccordement d'aspiration (8) au point le plus bas de l'espace intérieur (100).

17. Dispositif selon l'une des revendications 11 à 16,
caractérisé par
un récipient collecteur (12) destiné au condensat provenant des conduites d'amenée (24, 27) de vapeur de processus et de l'espace d'enveloppe (30), et muni d'une conduite de remise en circulation (23) et d'une pompe (31) pour la remise en circulation du condensat dans le circuit d'amenée de la vapeur de processus ainsi que d'un raccordement d'eau fraîche.

18. Dispositif selon l'une des revendications 11 à 17,
caractérisé en ce que
le condenseur (67) est refroidi par air.

19. Dispositif selon l'une des revendications 11 à 18,
caractérisé en ce que
le séparateur de liquide eSt conçu comme un cyclone (74).

20. Dispositif selon l'une des revendications 11 à 19,
caractérisé en ce que
les séparateurs de liquide sont refroidis à l'air.

21. Dispositif selon l'une des revendications 11 à 20,
caractérisé en ce que
pour la séparation des germes et/ou la séparation des matières solides, on monte deux filtres (34, 35) en série dont le préfiltre (34) dispose d'une grandeur de passage d'environ 1,2 µm et le filtre stérile (35), placé à la suite, d'une grandeur de passage d'environ 0,2 µm.

22. Dispositif selon la revendication 21,
caractérisé en ce qu'
une amenée de vapeur de processus (39) débouche directement entre le préfiltre (34) et le filtre stérile (35).

23. Dispositif selon l'une des revendications 11 à 22,
caractérisé en ce que
la conduite (33) débouche, du point le plus bas de l'espace intérieur (100) dans la conduite d'aspiration (32) du raccordement supérieur (9) de l'espace intérieur, derrière les filtres (34, 35) et devant le condenseur (67).

24. Dispositif selon l'une de revendications 11 à 23,
caractérisé en ce que
le laveur de gaz se compose de deux zones disposées l'une au-dessus de l'autre sensiblement a la verticale, à travers lesquelles sont guidés les gaz du bas vers le haut, une pompe d'aspiration étant disposée dans le sens de passage après la laveur de gaz et un liquide de circulation s'écoulant en circuit à travers le laveur de gaz essentiellement à contre-courant, c'est-à-dire du haut vers le bas, le laveur de gaz
- présentant dans la zone la plus basse une accumulation de liquide de circulation dans une cuvette,
- le gaz à purifier étant introduit dans la zone se trouvant au-dessus et un brouillard très fin étant généré à partir d'un liquide de lavage non souillé à l'aide de buses à brouillard à ultrasons,
- les substances contenues dans l'air étant éliminées dans une couche de séparation, disposée au-dessus, composée d'une matière solide de grande surface, telle que la roche volcanique ou similaires, et arrosée de liquide de circulation par le haut au moyen d'un dispositif de pulvérisation,
- les parts liquides des gaz de passage évacués étant retenues dans une couche pare-gouttes, disposée au dessus du dispositif de pulvérisation,
- le liquide de circulation, utilisé dans le dispositif de pulvérisation, étant prélevé du liquide de circulation accumulé au fond du laveur.

25. Dispositif selon la revendication 24,
caractérisé en ce que dans le circuit du liquide de lavage à l'extérieur du laveur de gaz, est disposé un accumulateur externe intermédiaire pour le liquide de lavage qui présente une ouverture d'aération ainsi qu'une arrivée d'eau fraîche.

26. Dispositif selon l'une des revendications 11 à 25,
caractérisé en ce que
les pompes à vide présentent un dispositif de chauffage.

27. Dispositif selon l'une des revendications 11 à 26,
caractérisé en ce que la station génératrice est logée à l'intérieur d'un conteneur mobile.
